(19) Europäisches Patentamt
European Patent Office
Office européen des brevets

(11) Veröffentlichungsnummer : **0 118 069**
**B1**

(12) # EUROPÄISCHE PATENTSCHRIFT

(45) Veröffentlichungstag der Patentschrift : **30.12.86**

(21) Anmeldenummer : **84101712.2**

(22) Anmeldetag : **20.02.84**

(51) Int. Cl.⁴ : **A 01 N 43/64**, A 01 N 43/50// C07D249/08, C07D233/60, C07D403/06, C07D405/06, C07C49/00

(54) **Fungizide Mittel 1,3-Diazolyl-propanole enthaltend.**

(30) Priorität : **02.03.83 DE 3307216**

(43) Veröffentlichungstag der Anmeldung : **12.09.84 Patentblatt 84/37**

(45) Bekanntmachung des Hinweises auf die Patenterteilung : **30.12.86 Patentblatt 86/52**

(84) Benannte Vertragsstaaten : **AT BE CH DE FR GB IT LI NL SE**

(56) Entgegenhaltungen :
**EP-A- 0 044 605**
**EP-A- 0 061 051**
**EP-A- 0 069 442**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Elbe, Hans-Ludwig, Dr.**
**Dasnöckel 59**
**D-5600 Wuppertal 11 (DE)**
Erfinder : **Holmwood, Graham, Dr.**
**Krutscheider Weg 105**
**D-5600 Wuppertal 11 (DE)**
Erfinder : **Regel, Erik, Dipl.-Ing.**
**Untere Bergerheide 26**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Reinecke, Paul, Dr.**
**Lessingstrasse 11**
**D-5090 Leverkusen 3 (DE)**
Erfinder : **Brandes, Wilhelm, Dr.**
**Eichendorffstrasse 3**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von substituierten 1,3-Diazolyl-2-propanolen zur Bekämpfung von phytopathogenen Pilzen.

Es ist bereits bekannt geworden, daß bestimmte Diazolyl-Derivate, wie beispielsweise 2-(4-Chlorphenyl)-1,3-di-(1,2,4-triazol-1-yl)-2-propanol, fungizide Eigenschaften aufweisen (vergleichen EP-A 0 044 605). Die Wirkung dieser Verbindungen ist jedoch, insbesondere bei niedrigen Aufwandmengen und -konzentrationen, nicht immer ganz befriedigend.

Die allgemeine Formel, die in der EP-A 0 044 605 enthalten ist, umfaßt unter anderem auch solche Verbindungen, in denen das Carbinol-Kohlenstoffatom sowohl mit zwei Azolylmethyl-Gruppen als auch mit einem gegebenenfalls substituierten Aralkyl-Rest verbunden ist. Es werden jedoch keine derartigen Stoffe offenbart, in denen das zur Carbinol-Gruppe benachbarte Kohlenstoffatom des Aralkyl-Restes zweifach durch Alkyl substituiert ist.

Es wurde nun gefunden, daß sich die substituierten 1,3-Diazolyl-2-propanole der allgemeinen Formel

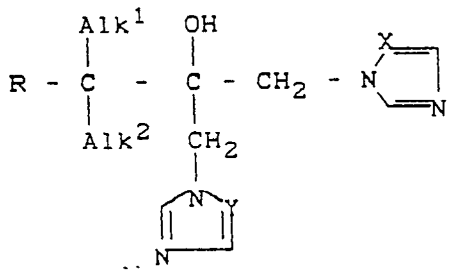

(I)

in welcher

Alk[1] für geradkettiges oder verzweigtes Alkyl mit 1 bis 4 Kohlenstoffatomen steht,

Alk[2] für geradkettiges oder verzweigtes Alkyl mit 1 bis 4 Kohlenstoffatomen steht,

Alk[1] und Alk[2] gemeinsam für einen 3- bis 7-gliedrigen cycloaliphatischen Ring stehen,

X für ein Stickstoffatom oder die CH-Gruppe steht,

Y für ein Stickstoffatom oder die CH-Gruppe steht,

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten genannt seien : Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoffatomen und 1 bis 5 gleichen oder verschiedenen Halogenatomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen im Alkylteil, Alkoximinoalkyl mit 1 bis 4 Kohlenstoffatomen in jedem Alkylteil sowie jeweils gegebenenfalls durch Halogen und/oder Alkyl mit 1 bis 2 Kohlenstoffatomen substituiertes Phenyl, Phenoxy, Benzyl und Benzyloxy,

sowie deren physiologisch verträgliche Säureadditions-Salze und Metallsalz-Komplexe gut zur Bekämpfung von phytopathogenen Pilzen verwenden lassen.

Überraschenderweise zeigen die erfindungsgemäß zu verwendenden substituierten 1,3-Diazolyl-2-propanole der Formel (I) eine bessere fungizide Wirksamkeit als die aus dem Stand der Technik bereits bekannten Diazolyl-Derivate, wie beispielsweise 2-(4-Chlorphenyl)-1,3-di-(1,2,4-triazol-1-yl)-2-propanol, welche konstitutionell und wirkungsmäßig naheliegende Verbindungen sind. Die erfindungsgemäße Verwendung dieser Stoffe stellt somit eine Bereicherung der Technik dar.

Die erfindungsgemäß zu verwendenden substituierten 1,3-Diazolyl-2-propanole sind durch die Formel (I) allgemein definiert.

Bevorzugt sind diejenigen Verbindungen der Formel (I), in denen

Alk[1] für geradkettiges oder verzweigtes Alkyl mit 1 bis 4 Kohlenstoffatomen steht,

Alk[2] für geradkettiges oder verzweigtes Alkyl mit 1 bis 4 Kohlenstoffatomen steht,

Alk[1] und Alk[2] gemeinsam mit dem Kohlenstoffatom an das sie gebunden sind, für Cyclobutyl, Cyclopentyl oder Cyclohexyl stehen,

X für ein Stickstoffatom oder die CH-Gruppe steht,

Y für ein Stickstoffatom oder die CH-Gruppe steht, und

R für jeweils gegebenenfalls im Phenylteil einfach bis zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten genannt seien : Fluor, Chlor, Brom, Methyl, Isopropryl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie jeweils gegebenenfalls

durch Fluor, Chlor, Methyl substituiertes Phenyl, Phenoxy, Benzyl und Benzyloxy.

Ganz besonders bevorzugt sind diejenigen Verbindungen der Formel (I), in denen

$Alk^1$ für Methyl oder Ethyl steht,

$Alk^2$ für Methyl oder Ethyl steht,

$Alk^1$ und $Alk^2$ gemeinsam mit dem Kohlenstoffatom an das sie gebunden sind, für Cyclobutyl, Cyclopentyl oder Cyclohexyl stehen,

X für ein Stickstoffatom oder die CH-Gruppe steht,

Y für ein Stickstoffatom oder die CH-Gruppe steht, und

R für jeweils gegebenenfalls im Phenylteil einfach bis zweifach, gleich oder verschieden substituiertes Phenyl, Benzyl, Phenethyl, Phenoxymethyl, Phenoxyethyl, Phenylthiomethyl oder Phenylthioethyl steht, wobei als Substituenten genannt seien : Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl sowie jeweils gegebenenfalls durch Fluor, Chlor, Methyl substituiertes Phenyl, Phenoxy, Benzyl und Benzyloxy.

Ganz besonders bevorzugt sind außerdem diejenigen Verbindungen der Formel (I), in denen

$Alk^1$ für Methyl oder Ethyl steht,

$Alk^2$ für Methyl oder Ethyl steht,

$Alk^1$ und $Alk^2$ gemeinsam mit dem Kohlenstoffatom an das sie gebunden sind, für Cyclobutyl, Cyclopentyl oder Cyclohexyl stehen,

X für ein Stickstoffatom oder die CH-Gruppe steht,

Y für ein Stickstoffatom oder die CH-Gruppe steht, und

R für jeweils gegebenenfalls im Phenylteil einfach bis zweifach, gleich oder verschieden substituiertes Phenoxy, Phenylthio, Benzyloxy oder Benzylthio steht, wobei als Substituenten genannt seien : Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroxyiminoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy.

Bevorzugte erfindungsgemäße Verbindungen sind auch Additionsprodukte aus Säuren und denjenigen substituierten 1,3-Diazolyl-2-propanolen der Formel (I), in denen die Substituenten $Alk^1$, $Alk^2$, X, Y und R die Bedeutungen haben, die bereits für diese Substituenten genannt wurden.

Zu den Säuren, die addiert werden können, gehören vorzugsweise Halogenwasserstoffsäuren, wie z. B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VII. Nebengruppe und denjenigen substituierten 1,3-Diazolyl-2-propanolen der Formel (I), in denen die Substituenten $Alk^1$, $Alk^2$, X, Y und R die Bedeutungen haben, die bereits für diese Substituenten genannt wurden.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure.

Die erfindungsgemäß zu verwendenden Wirkstoffe sind Gegenstand der DE-A 33 07 217 bzw. der EP-A 0 118 070 und werden erhalten, indem man 2-Azolylmethyl-oxirane der Formel

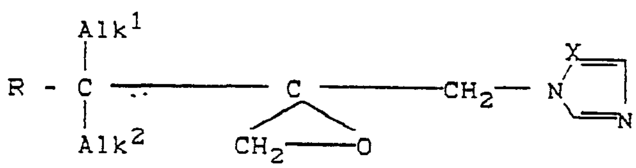

(II)

in welcher $Alk^1$, $Alk^2$, R und X die oben angegebene Bedeutung haben, mit Azolen der Formel

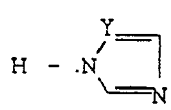

(III)

in welcher Y die oben angegebene Bedeutung hat, in Gegenwart eines inerten organischen Verdünnungsmittels, wie beispielsweise Alkohole, und gegebenenfalls in Gegenwart einer Base, wie beispielsweise

Alkalimetallalkoholate, bei Temperaturen zwischen 60 und 150 °C umsetzt.

Die Oxirane der Formel (II) sind bekannt (vergleiche DE-A 3 111 238, DE-A 32 02 601 und DE-A 32 37 400) und können, wie auch in diesen Anmeldungen beschrieben, in allgemein bekannter Art und Weise erhalten werden, indem man Azolylketone der Formel

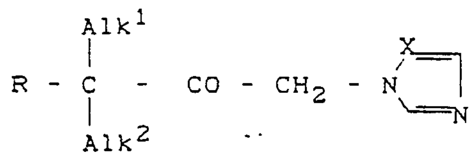 (IV)

in welcher Alk[1], Alk[2], R und X die oben angegebene Bedeutung haben, entweder

a) mit Dimethyloxosulfonium-methylid der Formel

$$\overset{\delta+\ \delta-}{(CH_3)_2SOCH_2} \quad (V)$$

in an sich bekannter Weise in Gegenwart eines Verdünnungsmittels, wie beispielsweise Dimethylsulfoxid, bei Temperaturen zwischen 20 °C und 80 °C umsetzt (vgl. hierzu die Angaben in J. Am. Chem. Soc. *87*, 1363-1364 (1965)), oder

b) mit Trimethylsulfonium-methylsulfat der Formel

$$[(CH_3)_3S^{\oplus}]\ CH_3SO_4^{\ominus} \quad (VI)$$

in an sich bekannter Weise in Gegenwart eines inerten organischen Lösungsmittels, wie z. B. Acetonitril, und in Gegenwart einer Base, wie z. B. Natriummethylat, bei Temperaturen zwischen 0 °C bis 60 °C, vorzugsweise bei Raumtemperatur, umsetzt (vgl. auch die Angaben in Heterocycles 8, 397 (1977)).

Die so erhaltenen Oxirane der Formel (II) können gegebenenfalls ohne Isolierung direkt weiter umgesetzt werden.

Die Azolyl-ketone der Formel (IV) sind bekannt (vergleiche DE-A 30 48 266 und DE-A 31 11 238) bzw. lassen sie sich nach im Prinzip bekannten Verfahren herstellen.

Die Säureadditionssalze der Verbindungen der Formel (I) können in einfacher Weise nach üblichen Salzbildungmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Cochliobolus-Arten, wie Cochliobolus sativus ; von Botrytis-Arten, wie gegen den Erreger des Grauschimmels an Erdbeeren und Trauben (Botrytis cinerea) ; ferner auch zur Bekämpfung von Mehltau, Rost, Septoria, Cercosporella und Pyrenophora teres an Getreide und gegen Venturia-Arten im Obstbau sowie von Reiskrankheiten, wie Pyricularia oryzae und Pellicularia sasakii, eingesetzt werden.

Außerdem zeigen die erfindungsgemäßen Verbindungen eine gute fungizide *in vitro* Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder

festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

Herstellungsbeispiele

Beispiel 1

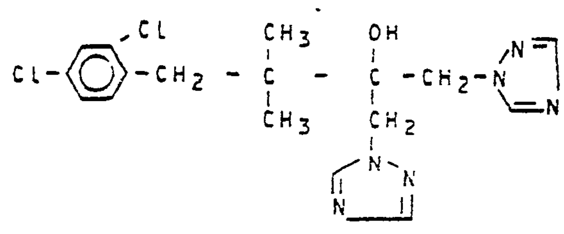

Zu einer Lösung von 0,11 g (47 mMol) Natrium in 30 ml n-Propanol werden bei Raumtemperatur unter Rühren 3,7 g (52,6 mMol) 1,2,4-Triazol gegeben. Man erhitzt auf Rückflußtemperatur und versetzt mit einer Lösung von 15,4 g (47 mMol) 2-(2,4-Dichlorphenyl-tert.-butyl)-2-(1,2,4-triazol-1-yl-methyl)-oxiran in 20 ml n-Propanol. Das Reaktionsgemisch wird 15 Stunden unter Rückfluß erhitzt, danach abgekühlt und auf Wasser gegeben. Man extrahiert mit Methylenchlorid, trocknet die organische Phase über

Natriumsulfat und engt ein. Der Rückstand wird säulenchromatographisch (Kieselgel ; Essigester : Cyclohexan = 3 : 1) gereinigt. Man erhält 3,5 g (18,8 % der Theorie) 4-(2,4-Dichlorphenyl)-3,3-dimethyl-2-(1,2,4-triazol-1-yl-methyl)-1-(1,2,4-triazol-1-yl)-2-butanol vom Schmelzpunkt 126 °C.

Herstellung des Ausgangsproduktes

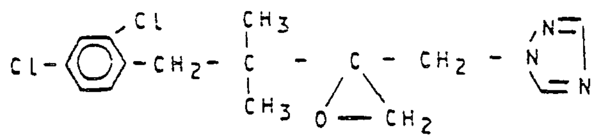

15,7 g (71,2 mMol) Trimethylsulfoxoniumiodid werden unter Stickstoffatmosphäre in 16 g Dimethylsulfoxid gelöst. Unter Kühlung wird bei Raumtemperatur mit 9,4 g (71,2 mMol) Kalium-tert.-butylat versetzt. Man läßt 6 Stunden nachrühren und versetzt anschließend mit einer Lösung von 20 g (64,1 mMol) 4-(2,4-Dichlorphenyl)-3,3-dimethyl-1-(1,2,4-triazol-1-yl)-2-butanon in 30 ml Tetrahydrofuran. Man läßt das Reaktionsgemisch 15 Stunden bei Raumtemperatur und 4 Stunden unter Rückfluß rühren, kühlt ab und gibt auf Wasser. Man extrahiert mit Methylenchlorid, trocknet die organische Phase über Natriumsulfat und engt im Vakuum ein. Man erhält 15,4 g (73,7 % der Theorie) 2-(2,4-Dichlorphenyl-tert.-butyl)-2-(1,2,4-triazol-1-yl-methyl)-oxiran vom Brechungsindex $n_D^{20}$ = 1,553 9.

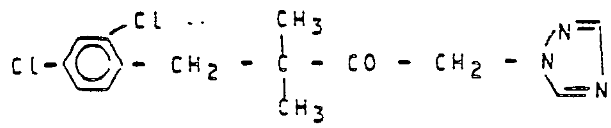

30 g (0,09 Mol) 1-Brom-4-(2,4-dichlorphenyl)-3,3-dimethyl-2-butanon, 12,4 g (0,18 Mol) 1,2,4-Triazol und 24,8 g (0,18 Mol) Kaliumcarbonat werden in 300 ml Aceton 6 Stunden unter Rückfluß erhitzt. Danach läßt man abkühlen, saugt ab und engt die Mutterlauge im Vakuum ein. Der Rückstand wird in Methylenchlorid aufgenommen, mit Wasser gewaschen, über Natriumsulfat getrocknet und im Vakuum eingeengt. Der Rückstand wird aus Diethylether umkristallisiert. Man erhält 12,8 g (45,6 % der Theorie) 4-(2,4-Dichlorphenyl)-3,3-dimethyl-1-(1,2,4-triazol-1-yl)-2-butanon vom Schmelzpunkt 85 °C.

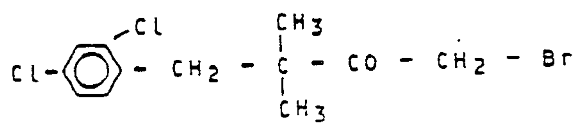

Zu einer Lösung von 64,5 g (0,26 Mol) 4-(2,4-Dichlorphenyl)-3,3-dimethyl-2-butanon in 600 ml Chloroform läßt man bei Raumtemperatur langsam 13,4 ml (0,26 Mol) Brom zutropfen. Die Reaktionslösung wird 1 Stunde bei Raumtemperatur nachgerührt. Anschließend wird durch Abdestillieren des Lösungsmittels eingeengt. Man erhält 84,3 g (100 % der Theorie) rohes 1-Brom-4-(2,4-dichlorphenyl)-3,3-dimethyl-2-butanon als Öl, das direkt weiter umgesetzt wird.

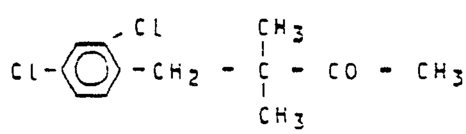

172 g (2 Mol) Methylisopropylketon, 391 g (2 Mol) 2,4-Dichlorbenzylchlorid, 20 g Tetrabutylammoniumbromid und 140 g (2,5 Mol) gepulvertes Kaliumhydroxid werden 15 Stunden unter Rückfluß erhitzt. Man läßt abkühlen und versetzt mit Wasser. Die organische Phase wird abgetrennt, über Natriumsulfat getrocknet und fraktioniert destilliert. Man erhält 129 g (26,4 % der Theorie) vom Siedepunkt 90-95 °C/0,05 mbar.

In entsprechender Weise werden die folgenden Verbindungen der allgemeinen Formel

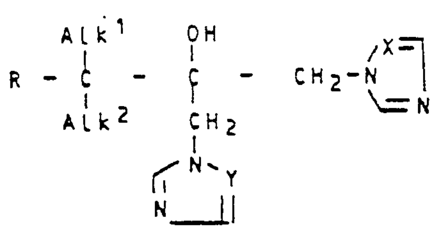

(I)

erhalten :

| Bsp. Nr. | R | $Alk^1$ | $Alk^2$ | X | Y | Schmelzpunkt [°C]; Brechungsindex [$n_D^{20}$]; |
|---|---|---|---|---|---|---|
| 2 | 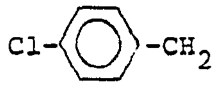 | $CH_3$ | $CH_3$ | N | N | 132 |
| 3 | 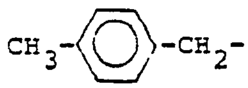 | $CH_3$ | $CH_3$ | N | N | 124 |
| 4 | 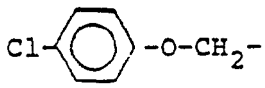 | $CH_3$ | $CH_3$ | N | N | 119-28 |
| 5 | 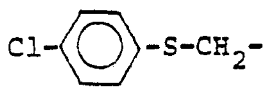 | $CH_3$ | $CH_3$ | N | N | 124 |
| 6 | 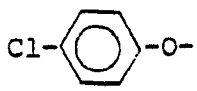 | $CH_3$ | $CH_3$ | N | N | 128 |
| 7 | 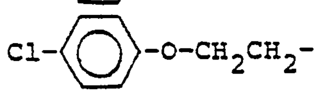 | $CH_3$ | $CH_3$ | N | N | 1,5460 |
| 8 | 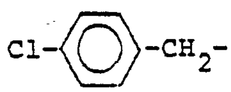 | $CH_3$ | $CH_3$ | CH | N | 220 (xHCl) |
| 9 | 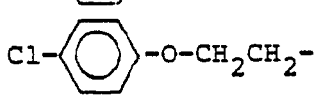 | $CH_3$ | $CH_3$ | CH | N | 1,5478 |
| 10 | 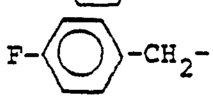 | $CH_3$ | $CH_3$ | N | N | 129 |
| 11 | 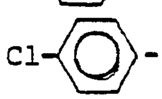 | 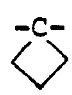 | | N | N | 78 |
| 12 | 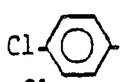 | $CH_3$ | $CH_3$ | N | N | 48 |
| 13 | 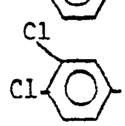 | $CH_3$ | $CH_3$ | N | N | 138 |
| 14 | 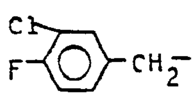 | $CH_3$ | $CH_3$ | N | N | 147 |
| 15 | 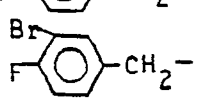 | $CH_3$ | $CH_3$ | N | N | 144 |

## Verwendungsbeispiele

In den nachfolgenden Beispielen wird die nachstehend angegebene Verbindung als Vergleichssubstanz eingesetzt :

(A) 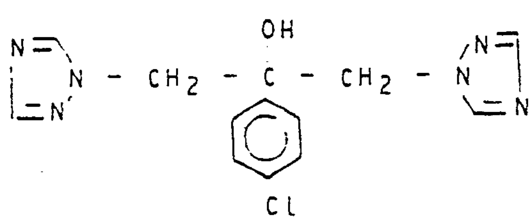

Beispiel A

Cochliobolus sativus-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 2, 3, 1 und 5.

Tabelle A

Cochliobolus sativus-Test(Gerste)/protektiv

| Wirkstoff | Wirkstoffkonzentration in der Spritzbrühe in Gew.-% | Krankheitsbefall in % der unbehandelten Kontrolle |
|---|---|---|
| 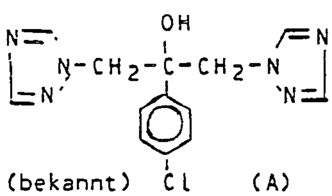 (bekannt) (A) | 0,025 | 100 |
| 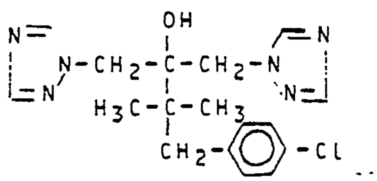 (2) | 0,025 | 0,0 |
| 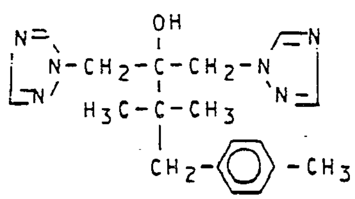 (3) | 0,025 | 12,5 |
| 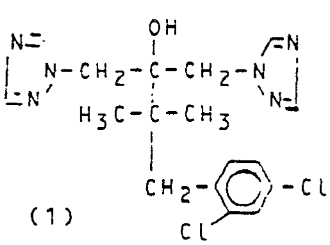 (1) | 0,025 | 0,0 |

(Fortsetzung)

| Wirkstoff | Wirkstoffkonzentration in der Spritzbrühe in Gew.-% | Krankheitsbefall in % der unbehandelten Kontrolle |
|---|---|---|
| 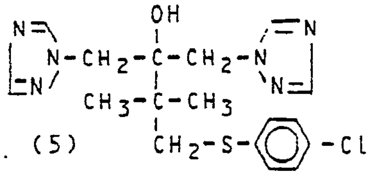 (5) | 0,025 | 21,2 |

Beispiel B

Botrytis-Test (Bohne)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z. B. die Verbindungen gemäß folgender Herstellungsbeispiele : 2, 3 und 7.

Tabelle B

Botrytis-Test (Bohne)/protektiv

| Wirkstoff | Befall in % bei einer Wirkstoffkonzentration von 250 ppm |
|---|---|
| 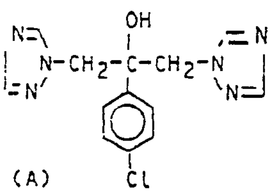 (A) (bekannt) | 100 |
| 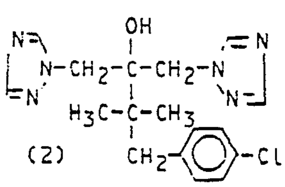 (2) | 75 |

(Fortsetzung)

| Wirkstoff | Befall in % bei einer Wirkstoffkonzentration von 250 ppm |
|---|---|
| 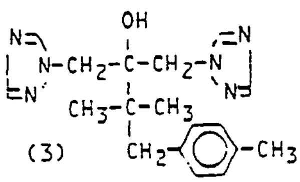 (3) | 26 |
| 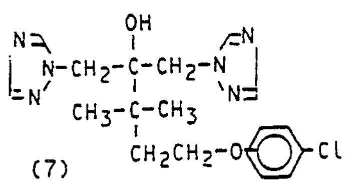 (7) | 0 |

## Patentansprüche

1. Verwendung von 1,3-Diazolyl-2-propanolen der Formel

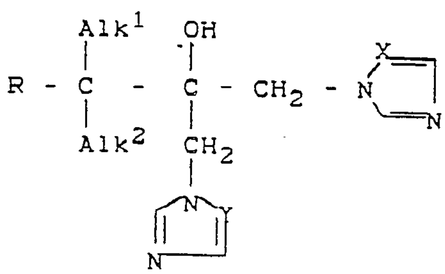

(I)

in welcher

Alk[1] für geradkettiges oder verzweigtes Alkyl mit 1 bis 4 Kohlenstoffatomen steht,

Alk[2] für geradkettiges oder verzweigtes Alkyl mit 1 bis 4 Kohlenstoffatomen steht,

Alk[1] und Alk[2] gemeinsam für einen 3- bis 7-gliedrigen cycloaliphatischen Ring stehen,

X für ein Stickstoffatom oder die CH-Gruppe steht,

Y für ein Stickstoffatom oder die CH-Gruppe steht,

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten genannt seien : Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoffatomen und 1 bis 5 gleichen oder verschiedenen Halogenatomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen im Alkylteil, Alkoximinoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy,

sowie von deren physiologisch verträglichen Säureadditions-Salzen und Metallsalzkomplexen zur Bekämpfung von phytopathogenen Pilzen.

2. Verwendung gemäß Anspruch 1, dadurch gekennzeichnet, daß man Verbindungen der Formel (I), in welcher

$Alk^1$ für geradkettiges oder verzweigtes Alkyl mit 1 bis 4 Kohlenstoffatomen steht,
$Alk^2$ für geradkettiges oder verzweigtes Alkyl mit 1 bis 4 Kohlenstoffatomen steht,
$Alk^1$ und $Alk^2$ gemeinsam mit dem Kohlenstoffatom an das sie gebunden sind, für Cyclobutyl, Cyclopentyl oder Cyclohexyl stehen,
X für ein Stickstoffatom oder die CH-Gruppe steht,
Y für ein Stickstoffatom oder CH-Gruppe steht, und
R für jeweils gegebenenfalls im Phenylteil einfach bis zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten genannt seien : Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy,
oder deren physiologisch verträgliche Säureadditionssalze und Metallsalzkomplexe einsetzt.

3. Verwendung gemäß Anspruch 1, dadurch gekennzeichnet, daß man Verbindungen der Formel (I), in welcher
$Alk^1$ für Methyl oder Ethyl steht,
$Alk^2$ für Methyl oder Ethyl steht,
$Alk^1$ und $Alk^2$ gemeinsam mit dem Kohlenstoffatom an das sie gebunden sind, für Cyclobutyl, Cyclopentyl oder Cyclohexyl stehen,
X für ein Stickstoffatom oder die CH-Gruppe steht,
Y für ein Stickstoffatom oder die CH-Gruppe steht, und
R für jeweils gegebenenfalls im Phenylteil einfach bis zweifach, gleich oder verschieden substituiertes Phenyl, Benzyl, Phenetyl, Phenoxymethyl, Phenoxyethyl, Phenylthiomethyl oder Phenylthioethyl steht, wobei als Substituenten genannt seien : Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy,
oder deren physiologisch verträgliche Säureadditionssalze und Metallsalzkomplexe einsetzt.

4. Verwendung gemäß Anspruch 1, dadurch gekennzeichnet, daß man Verbindungen der Formel (I), in welcher
$Alk^1$ für Methyl oder Ethyl steht,
$Alk^2$ für Methyl oder Ethyl steht,
$Alk^1$ und $Alk^2$ gemeinsam mit dem Kohlenstoffatom, an das sie gebunden sind, für Cyclobutyl, Cyclopentyl oder Cyclohexyl stehen,
X für ein Stickstoffatom oder die CH-Gruppe steht,
Y für ein Stickstoffatom oder die CH-Gruppe steht, und
R für jeweils gegebenenfalls im Phenylteil einfach bis zweifach, gleich oder verschieden substituiertes Phenoxy, Phenylthio, Benzyloxy oder Benzylthio steht, wobei als Substituenten genannt seien : Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy,
oder deren physiologisch verträglichen Säureadditionssalze und Metallsalzkomplexe einsetzt.

**Claims**

1. Use of 1,3-diazolyl-2-propanols of the formula

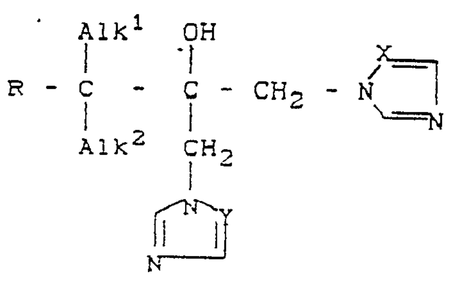

(I)

in which
$Alk^1$ represents straight-chain or branched alkyl with 1 to 4 carbon atoms,
$Alk^2$ represents straight-chain or branched alkyl with 1 to 4 carbon atoms,

$Alk^1$ and $Alk^2$ together represent a 3- to 7-membered cycloaliphatic ring,

X represents a nitrogen atom or the CH group,

Y represents a nitrogen atom or the CH group,

R represents phenyl, phenylalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, the substituents which may be mentioned being : halogen, alkyl with 1 to 4 carbon atoms, alkoxy and alkylthio with in each case 1 to 4 carbon atoms, halogenoalkyl and halogenoalkoxy and halogenoalkylthio with in each case 1 to 2 carbon atoms and 1 to 5 identical or different halogen atoms, nitro, cyano, hydroxyl, hydroxycarbonyl, alkoxycarbonyl with 1 to 4 carbon atoms in the alkyl part, hydroximinoalkyl with 1 to 4 carbon atoms in the alkyl part, alkoximinoalkyl with 1 to 4 carbon atoms in each alkyl part and phenyl, phenoxy, benzyl and benzyloxy, in each case optionally substituted by halogen and/or alkyl with 1 to 2 carbon atoms,

and their physiologically acceptable acid addition salts and metal salt complexes for combating phytopathogenic fungi.

2. Use according to Claim 1, characterised in that compounds of the formula (I) in which

$Alk^1$ represents straight-chain or branched alkyl with 1 to 4 carbon atoms,

$Alk^2$ represents straight-chain or branched alkyl with 1 to 4 carbon atoms,

$Alk^1$ and $Alk^2$, together with the carbon atom to which they are bonded, represent cyclobutyl, cyclopentyl or cyclohexyl,

X represents a nitrogen atom or the CH group,

Y represents a nitrogen atom or CH group, and

R represents phenyl, benzyl, phenethyl, phenoxy, phenylthio, phenoxymethyl, phenoxyethyl, phenylthiomethyl, phenylthioethyl, benzyloxy or benzylthio, each of which is optionally mono- to disubstituted in the phenyl part by identical or different substituents, substituents which may be mentioned being : fluorine, chlorine, bromine, methyl, isopropyl, tert.-butyl, methoxy, methylthio, trifluoromethyl, trifluoromethoxy, trifluoromethylthio, nitro, cyano, hydroxyl, hydroxycarbonyl, methoxycarbonyl, ethoxycarbonyl, hydroximinomethyl, 1-hydroximinoethyl, methoximinomethyl, 1-methoximinoethyl, and phenyl, phenoxy, benzyl and benzyloxy, in each case optionally substituted by fluorine, chlorine or methyl,

or their physiologically acceptable acid addition salts and metal salt complexes, are used.

3. Use according to Claim 1, characterised in that compounds of the formula (I) in which

$Alk^1$ represents methyl or ethyl,

$Alk^2$ represents methyl or ethyl,

$Alk^1$ and $Alk^2$, together with the carbon atom to which they are bonded, represent cyclobutyl, cyclopentyl or cyclohexyl,

X represents a nitrogen atom or the CH group,

Y represents a nitrogen atom or the CH group, and

R represents phenyl, benzyl, phenethyl, phenoxymethyl, phenoxyethyl, phenylthiomethyl or phenylthioethyl, each of which is optionally mono- to disubstituted in the phenyl part by identical or different substituents, the substituents which may be mentioned being : fluorine, chlorine, bromine, methyl, isopropyl, tert.-butyl, methoxy, methylthio, trifluoromethyl, trifluoromethoxy, trifluoromethylthio, nitro, cyano, hydroxyl, hydroxycarbonyl, methoxycarbonyl, ethoxycarbonyl, hydroximinomethyl, 1-hydroximinoethyl, methoximinomethyl, 1-methoximinoethyl, and phenyl, phenoxy, benzyl and benzyloxy, in each case optionally substituted by fluorine, chlorine or methyl,

or their physiologically acceptable acid addition salts and metal salt complexes.

4. Use according to Claim 1, characterised in that compounds of the formula (I) in which

$Alk^1$ represents methyl or ethyl,

$Alk^2$ represents methyl or ethyl,

$Alk^1$ and $Alk^2$, together with the carbon atom to which they are bonded, represent cyclobutyl, cyclopentyl or cyclohexyl,

X represents a nitrogen atom or the CH group,

Y represents a nitrogen atom or the CH group, and

R represents phenoxy, phenylthio, benzyloxy or benzylthio, each of which is optionally mono- to disubstituted in the phenyl part by identical or different substituents, the substituents which may be mentioned being : fluorine, chlorine, bromine, methyl, isopropyl, tert.-butyl, methoxy, methylthio, trifluoromethyl, trifluoromethoxy, trifluoromethylthio, nitro, cyano, hydroxyl, hydroxycarbonyl, methoxycarbonyl, ethoxycarbonyl, hydroximinomethyl, 1-hydroximinoethyl, methoximinomethyl, 1-methoximinoethyl, and phenyl, phenoxy, benzyl and benzyloxy, in each case optionally substituted by fluorine, chlorine or methyl,

or their physiologically acceptable acid addition salts and metal salt complexes, are used.

**Revendications**

1. Utilisation de 1,3-diazolyl-2-propanols de formule :

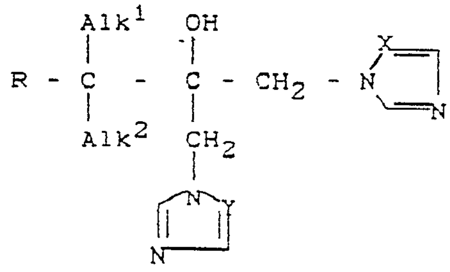

(I)

dans laquelle

Alk[1] représente un alcoyle à chaîne droite ou ramifiée ayant 1 à 4 atomes de carbone,

Alk[2] un alcoyle à chaîne droite ou ramifiée ayant 1 à 4 atomes de carbone,

Alk[1] et Alk[2] représentent ensemble un noyau cycloaliphatique ayant 3 à 7 chaînons,

X un atome d'azote ou le groupe CH,

Y un atome d'azote ou le groupe CH,

R un phényle, phénylalcoyle ayant 1 à 4 atomes de carbone dans la portion alcoyle, phénoxy, phénylthio, phénoxyalcoyle ayant 1 à 4 atomes de carbone dans la portion alcoyle, phénylthioalcoyle ayant 1 à 4 atomes de carbone dans la portion alcoyle, benzyloxy et benzylthio éventuellement substitués chacun dans la portion phényle une à trois fois, de manière identique ou différente, en mentionnant comme substituants : halogène, alcoyle ayant 1 à 4 atomes de carbone, alcoxy et alcoylthio ayant chacun 1 à 4 atomes de carbone, halogénoalcoyle et halogénoalcoxy de même que halogénoalcoylthio ayant chacun 1 à 2 atomes de carbone et 1 à 5 atomes d'halogène identiques ou différents, nitro, cyano, hydroxy, hydroxycarbonyle, alcoxycarbonyle ayant 1 à 4 atomes de carbone dans la portion alcoyle, hydroximinoalcoyle ayant 1 à 4 atomes de carbone dans la portion alcoyle, alcoximinoalcoyle ayant 1 à 4 atomes de carbone dans chaque portion alcoyle, de même que phényle, phénoxy, benzyle et benzyloxy éventuellement substitués chacun par de l'halogène et/ou un alcoyle ayant 1 à 2 atomes de carbone, de même que de leurs sels d'addition d'acides et complexes avec des sels métalliques physiologiquement compatibles dans la lutte contre les champignons phytopathogènes.

2. Utilisation selon la revendication 1, caractérisée en ce qu'on utilise des composés de formule (I) dans laquelle

Alk[1] représente un alcoyle à chaîne droite ou ramifiée ayant 1 à 4 atomes de carbone,

Alk[2] un alcoyle à chaîne droite ou ramifiée ayant 1 à 4 atomes de carbone,

Alk[1] et Alk[2] conjointement avec l'atome de carbone auquel ils sont attachés représentent un cyclobutyle, cyclopentyle ou cyclohexyle,

X un atome d'azote ou le groupe CH,

Y un atome d'azote ou le groupe CH,

R un phényle, benzyle, phénéthyle, phénoxy, phénylthio, phénoxyméthyle, phénoxyéthyle, phénylthiométhyle, phénylthioéthyle, benzyloxy ou benzylthio éventuellement substitués chacun dans la portion phényle une à deux fois, de manière identique ou différente, en citant en tant que substituants : fluor, chlore, brome, méthyle, isopropyle, t-butyle, méthoxy, méthylthio, trifluorométhyle, trifluorométhoxy, trifluorométhylthio, nitro, cyano, hydroxy, hydroxycarbonyle, méthoxycarbonyle, éthoxycarbonyle, hydroximinométhyle, 1-hydroximinoéthyle, méthoximinométhyle, 1-méthoximinoéthyle, de même que phényle, phénoxy, benzyle et benzyloxy éventuellement substitués chacun par du fluor, du chlore, un méthyle,

ou leurs sels d'addition d'acides et complexes avec des sels métalliques physiologiquement compatibles.

3. Utilisation selon la revendication 1, caractérisée en ce qu'on utilise des composés de formule (I) dans laquelle

Alk[1] représente un méthyle ou éthyle,

Alk[2] un méthyle ou éthyle,

Alk[1] et Alk[2] conjointement avec l'atome de carbone auquel ils sont attachés représentent un cyclobutyle, cyclopentyle ou cyclohexyle,

X un atome d'azote ou le groupe CH,

Y un atome d'azote ou le groupe CH,

R un phényle, benzyle, phénéthyle, phénoxyméthyle, phénoxyéthyle, phénylthiométhyle ou phénylthioéthyle éventuellement substitués chacun dans la portion phényle une à deux fois, de manière identique ou différente, en citant comme substituants : fluor, chlore, brome, méthyle, isopropyle, t-butyle, méthoxy, méthylthio, trifluorométhyle, trifluorométhoxy, trifluorométhylthio, nitro, cyano, hydroxy, hydroxycarbonyle, méthoxycarbonyle, éthoxycarbonyle, hydroximinométhyle, 1-hydroximinoéthyle, méthoxyminométhyle, 1-méthoximinoéthyle, de même que phényle, phénoxy, benzyle et benzyloxy éventuellement substitués chacun par du fluor, du chlore, un méthyle,

ou leurs sels d'addition d'acides et complexes avec des sels métalliques physiologiquement compatibles.

4. Utilisation selon la revendication 1, caractérisée en ce qu'on utilise des composés de formule (I) dans laquelle

$Alk^1$ représente un méthyle ou éthyle,

$Alk^2$ un méthyle ou éthyle,

$Alk^1$ et $Alk^2$ conjointement avec l'atome de carbone auquel ils sont attachés représentent un cyclobotyle, cyclopentyle ou cyclohexyle,

X un atome d'azote ou le groupe CH,

Y un atome d'azote ou le groupe CH,

R un phénoxy, phénylthio, benzyloxy ou benzylthio éventuellement substitués chacun dans la portion phényle une à deux fois, de manière identique ou différente, en citant en tant que substituants : fluor, chlore, brome, méthyle, isopropyle, t-butyle, méthoxy, méthylthio, trifluorométhyle, trifluorométhoxy, trifluorométhylthio, nitro, cyano, hydroxy, hydroxycarbonyle, méthoxycarbonyle, éthoxycarbonyle, hydroximinométhyle, 1-hydroximinoéthyle, méthoximinométhyle, 1-méthoximinoéthyle, de même que phényle, phénoxy, benzyle et benzyloxy éventuellement substitués chacun par du fluor, du chlore, un méthyle,

ou leurs sels d'addition d'acides et complexes avec des sels métalliques physiologiquement compatibles.